# EUROPEAN PATENT SPECIFICATION

(11) **EP 3 925 711 B1**
(45) Date of publication and mention of the grant of the patent: **18.03.2026**
(21) Application number: 20784076.0
(22) Date of filing: 04.02.2020
(51) Int. Cl.: B09B 3/00, B09B 3/40, B09B 3/45, C05F 9/02, C05F 17/10, C05F 17/50, C12M 1/00

(54) **WASTE TREATMENT SYSTEM AND WASTE TREATMENT METHOD**
ABFALLBEHANDLUNGSSYSTEM UND VERFAHREN ZUR ABFALLBEHANDLUNG
SYSTÈME DE TRAITEMENT DE DÉCHETS ET PROCÉDÉ DE TRAITEMENT DE DÉCHETS

(30) Priority: 29.03.2019 JP 2019066030
(43) Date of publication of application: 22.12.2021
(73) Proprietor: Mitsubishi Heavy Industries, Ltd., Tokyo 100-8332 (JP)
(72) Inventor: YUKUMOTO, Atsuhiro, Tokyo 100-8332 (JP); NOMA, Akira, Tokyo 100-8332 (JP); SATOU, Jun, Tokyo 100-8332 (JP); AOKI, Yasumichi, Tokyo 100-8332 (JP); YAMAGUCHI, Daiki, Tokyo 100-8332 (JP); SEIKI, Yoshio, Tokyo 100-8332 (JP); KOBAYASHI, Kazuto, Tokyo 100-8332 (JP); OKINO, Susumu, Tokyo 100-8332 (JP); WAKUI, Satoshi, Tokyo 100-8332 (JP)
(74) Representative: Studio Torta S.p.A.
(86) International application number: PCT/JP2020/004007
(87) International publication number: WO 2020/202771

(56) References cited:
- WO-A1-2013/008907
- WO-A1-2013/046622
- WO-A1-2013/046622
- WO-A1-2013/046624
- WO-A1-2013/046624
- JP-A- 2010 279 255
- JP-A- 2010 279 255
- JP-A- 2010 284 120
- JP-A- 2014 143 945
- JP-A- 2015 217 345

## Description

### TECHNICAL FIELD

The present disclosure relates to a waste treatment system and a waste treatment method.

### BACKGROUND

Patent Document 1 discloses a method in which a raw material containing plant waste is hydrolyzed with steam and is separated into solid and liquid components to produce a biomass fuel from the solid component and a liquid fertilizer from the liquid component.

### Citation List

### Patent Literature

Patent Document 1: JP6190082B
Patent Document 2: WO2013/046622
Patent Document 3: WO2013/046624
Patent Document 4: JP2010279255
Patent Document 5: JP2015217345
Patent Document 6: JP2010284120

### SUMMARY

### Problems to be Solved

However, the method disclosed in Patent Document 1 has some problems, such as the inability to appropriately treat waste containing a large amount of nitrogen compounds such as proteins and to produce fermented substances from them.

In view of the above, an object of at least one embodiment of the present disclosure is to provide a waste treatment system and a waste treatment method whereby it is possible to increase a treatable waste range and appropriately produce a fermented substance using treated waste as the raw material.

### Solution to the Problems

According to the present invention there is provided a waste treatment system, comprising: a first hydrothermal treatment device for performing hydrothermal treatment of waste; a first solid-liquid separation device for separating a first reactant of the first hydrothermal treatment device into a first separated product which is a solid and a second separated product which is a liquid or slurry; a second hydrothermal treatment device for performing hydrothermal treatment of the first separated product; a second solid-liquid separation device for separating a second reactant of the second hydrothermal treatment device into a third separated product which is a solid and a fourth separated product which is a liquid or slurry; and a fermentation device for fermenting the second separated product and the fourth separated product, wherein a first temperature which is a temperature of the hydrothermal treatment in the first hydrothermal treatment device is a temperature that suppresses generation of a fermentation inhibitor by Maillard reaction, and a second temperature which is a temperature of the hydrothermal treatment in the second hydrothermal treatment device is a temperature that suppresses generation of a fermentation inhibitor including an aromatic compound, a carbide, or a precursor thereof from the first separated product.

Herein, the first hydrothermal treatment device, the second hydrothermal treatment device, the first solid-liquid separation device, the second solid-liquid separation device, and the fermentation device each may include multiple machines that have their functions. For example, the system may be provided with two first hydrothermal treatment devices and one second hydrothermal treatment device.

When waste containing a large amount of nitrogen compounds such as proteins is hydrothermally treated at a temperature of 150°C or higher, melanoidin is significantly generated by the Maillard reaction. Nitrogen-containing antioxidants such as melanoidin entering the fermentation device inhibit fermentation. With the above configuration, since the hydrothermal treatment in the first hydrothermal treatment device is performed at a temperature that suppresses the Maillard reaction, the generation of melanoidin in the first hydrothermal treatment device can be suppressed. Further, since the first solid-liquid separation device solubilizes or slurries nitrogen compounds such as proteins, which may cause melanoidin formation, and separates them as a liquid or slurry of the first reactant, the amount of nitrogen compounds in the raw material of the second hydrothermal treatment device can be reduced, and the generation of melanoidin due to the hydrothermal treatment in the second hydrothermal treatment device can be suppressed. Thus, it is possible to reduce the fermentation inhibition in the fermentation device. As a result, it is possible to increase a treatable waste range and appropriately produce a fermented substance using treated waste as the raw material.

In some embodiments, in the above configuration, a the first temperature is 120°C to 160°C, and the second temperature is 200°C to 240°C.
With this configuration, since the hydrothermal treatment in the second hydrothermal treatment device is performed at a higher temperature than the hydrothermal treatment in the first hydrothermal treatment device, persistent organic matter such as cellulose can be degraded to easily degradable organic matter such as saccharide and organic acid and supplied to the fermentation device, so that the fermentation time in the fermentation device can be shortened while suppressing the generation of melanoidin in the second hydrothermal treatment device. Herein, the terms persistent and easily degradable are used in the sense of whether it takes a long time or a short time for enzymes or bacteria in the fermentation device to decompose the raw material.

In some embodiments, in the above configuration , the waste treatment system comprises at least one sensor for detecting a concentration of the fermentation inhibitor contained in at least one of the second separated product or the fourth separated product. The at least one sensor includes at least one of: at least one colored substance sensor for detecting an index of a colored substance as the fermentation inhibitor; at least one pH sensor for detecting pH of at least one of the second separated product or the fourth separated product; at least one sodium sensor) for detecting a concentration of sodium as the fermentation inhibitor; at least one organic acid concentration sensor) for detecting a concentration of an organic acid as the fermentation inhibitor; or at least one ammonia concentration sensor for detecting a concentration of ammonia as the fermentation inhibitor.

With this configuration, since the index of melanoidin which is a colored substance in the liquid or the slurry flowing in the fermentation device can be detected, by adjusting the operating conditions of the waste treatment system, such as adjusting the temperature of the hydrothermal treatment in the first hydrothermal treatment device based on the detected index of melanoidin, the index of melanoidin in the liquid or the slurry flowing in the fermentation device can be reduced to reduce the risk of inhibiting fermentation.

A waste treatment method according at least one embodiment of the present disclosure comprises: a first hydrothermal treatment step of performing hydrothermal treatment of waste; a first solid-liquid separation step of separating a first reactant of the first hydrothermal treatment step into a first separated product which is a solid and a second separated product which is a liquid or slurry; a second hydrothermal treatment step of performing hydrothermal treatment of the first separated product; a second solid-liquid separation step of separating a second reactant of the second hydrothermal treatment step into a third separated product which is a solid and a fourth separated product which is a liquid or slurry; and a fermentation step of fermenting the second separated product and the fourth separated product, wherein a first temperature which is a temperature of the hydrothermal treatment in the first hydrothermal treatment device is a temperature that suppresses generation of a fermentation inhibitor by Maillard reaction, and a second temperature which is a temperature of the hydrothermal treatment in the second hydrothermal treatment device is a temperature that suppresses generation of a fermentation inhibitor including an aromatic compound, a carbide, or a precursor thereof from the first separated product.

When waste containing a large amount of nitrogen compounds such as proteins is hydrothermally treated at a temperature of 150°C or higher, melanoidin is generated by the Maillard reaction. Nitrogen-containing antioxidants such as melanoidin inhibit fermentation in the fermentation step. With the above configuration, since the hydrothermal treatment in the first hydrothermal treatment step is performed at a temperature that suppresses the Maillard reaction, the generation of melanoidin in the first hydrothermal treatment step can be suppressed. Further, since the first solid-liquid separation step solubilizes or slurries nitrogen compounds such as proteins, which may cause melanoidin formation, and transfers them into a liquid or slurry of the first reactant, the amount of nitrogen compounds in the raw material of the second hydrothermal treatment step can be reduced, and the generation of melanoidin due to the hydrothermal treatment in the second hydrothermal treatment step can be suppressed. Thus, it is possible to reduce the fermentation inhibition in the fermentation step. As a result, it is possible to increase a treatable waste range and appropriately produce a fermented substance using treated waste as the raw material.

(5) In some embodiments, in the above method (4), a temperature of the hydrothermal treatment in the first hydrothermal treatment step is lower than a temperature of the hydrothermal treatment in the second hydrothermal treatment step.

With this method, since the hydrothermal treatment in the second hydrothermal treatment step is performed at a higher temperature than the hydrothermal treatment in the first hydrothermal treatment step, persistent organic matter such as cellulose can be degraded to easily degradable organic matter and fermented, so that the fermentation time in the fermentation step can be shortened while suppressing the generation of melanoidin in the second hydrothermal treatment step.

In some embodiments, in the above method, the first hydrothermal treatment step and the second hydrothermal treatment step are performed in a same hydrothermal treatment device.

With this method, since the waste treatment method can be performed by one hydrothermal treatment device, the system for implementing the waste treatment method can be downsized.

In some embodiments, in the above method, the second hydrothermal treatment step is performed in a different hydrothermal treatment device from a hydrothermal device that has performed the first hydrothermal treatment step.

For example, at time t1, among four hydrothermal treatment devices with the same function, devices A and B discharge the product and feed the raw waste, device C performs the first hydrothermal treatment step, and device D performs the second hydrothermal treatment step. Then, at time t2, device A performs the first hydrothermal treatment step, device B performs the second hydrothermal treatment step, and devices C and D discharge the product and feed the raw waste. With this method, flexible operation is possible since the process can be performed in any device without choosing the raw material.

### Advantageous Effects

According to at least one embodiment of the present disclosure, since the hydrothermal treatment in the first hydrothermal treatment device is performed at a temperature that suppresses the Maillard reaction, the generation of melanoidin in the first hydrothermal treatment device can be suppressed. Further, since the first solid-liquid separation device solubilizes or slurries nitrogen compounds such as proteins, which may cause melanoidin formation, and transfers them into a liquid of the first reactant, the amount of nitrogen compounds in the raw material of the second hydrothermal treatment device can be reduced, and the generation of melanoidin due to the hydrothermal treatment in the second hydrothermal treatment device can be suppressed. Thus, it is possible to reduce the fermentation inhibition in the fermentation device. As a result, it is possible to increase a treatable waste range and appropriately produce a fermented substance using treated waste as the raw material.

### BRIEF DESCRIPTION OF DRAWINGS

FIG. 1 is a schematic configuration diagram of a waste treatment system according to a first embodiment of the present disclosure.
FIG. 2 is a schematic configuration diagram of a modified example of the waste treatment system according to the first embodiment of the present disclosure.
FIG. 3 is a schematic configuration diagram of the waste treatment system according to a second embodiment of the present disclosure.
FIG. 4 is a schematic configuration diagram of the waste treatment system according to a third embodiment of the present disclosure.
FIG. 5 is a schematic configuration diagram of a specific example of the waste treatment system according to the third embodiment of the present disclosure.
FIG. 6 is a schematic configuration diagram of the waste treatment system according to a fourth embodiment of the present disclosure.
FIG. 7 is a schematic configuration diagram of the waste treatment system according to a fifth embodiment of the present disclosure.
FIG. 8 is a schematic configuration diagram of the waste treatment system according to a sixth embodiment of the present disclosure.
FIG. 9 is a schematic configuration diagram of the waste treatment system according to a seventh embodiment of the present disclosure.

### DETAILED DESCRIPTION

Embodiments of the present invention will now be described in detail with reference to the accompanying drawings. However, the scope of the present invention is not limited to the following embodiments. It is intended that dimensions, materials, shapes, relative positions and the like of components described in the embodiments shall be interpreted as illustrative only and not intended to limit the scope of the present invention.

### (First Embodiment)

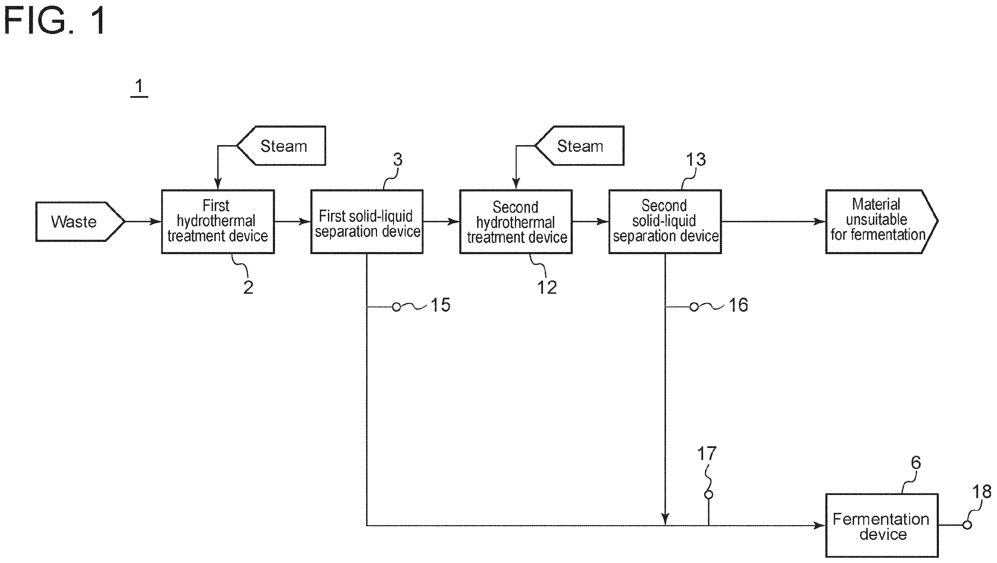

As shown in FIG. 1, a waste treatment system 1 according to the first embodiment of the present disclosure includes a first hydrothermal treatment device 2 for performing hydrothermal treatment of waste (first hydrothermal treatment step), a first solid-liquid separation device 3 for separating a first reactant, which is a reactant of the first hydrothermal treatment device 2, into a solid and a liquid (first solid-liquid separation step), a second hydrothermal treatment device for performing hydrothermal treatment of the solid of the first reactant (second hydrothermal treatment step), a second solid-liquid separation device for separating a second reactant, which is a reactant of the second hydrothermal treatment device, into a solid and a liquid (second solid-liquid separation step), and a fermentation device 6 for fermenting the liquid of the first reactant and the liquid of the second reactant (fermentation step).

The configuration of the first hydrothermal treatment device 2 is not limited, but may be, for example, a batch-type hydrothermal treatment device equipped with a container that can receive waste as it is from a vehicle or a plant that collects the waste, and configured to supply steam to the container. The configuration of the first solid-liquid separation device 3 is not limited, but may be any configuration that separates a liquid (liquid or slurry) passing through the first solid-liquid separation device 3 from a solid not passing through the first solid-liquid separation device 3, such as one that separates solid with at least a certain particle size. For example, it may be a screen, a mesh, a filter, a strainer, a filtration device, or a centrifugal separation device. The configurations of the second hydrothermal treatment device 12 and the second solid-liquid separation device 13 may be the same as or different from the configurations of the first hydrothermal treatment device 2 and the first solid-liquid separation device 3. In the following description, the liquids separated by the first solid-liquid separation device 3 and the second solid-liquid separation device 13 include not only liquid containing no fine solids, but also slurry containing fine solids.

The configuration of the fermentation device 6 is not limited, but may be any configuration that produces a valuable resource using the liquid of the first reactant and the liquid of the second reactant as the raw material through the biological action of microorganisms, bacteria, enzymes, etc. For example, it may be a methane fermentation device for producing biogas, an ethanol fermentation device for producing ethanol, an organic fertilizer production device for producing organic fertilizer, a feed production device for producing feed, or a combination of some of these. A pre-fermentation treatment facility such as an enzymatic saccharification tank, an acid fermentation tank, or a mixing and conditioning tank may be provided upstream of the fermentation device. Further, a facility for removing fine materials unsuitable for fermentation that cannot be removed by the first solid-liquid separation device 3 and the second solid-liquid separation device 13 may be provided. For example, this facility may be a sand separator which utilizes centrifugal force for separation.

Next, operation of the waste treatment system 1 according to the first embodiment of the present disclosure will be described.

As shown in FIG. 1, waste received by the first hydrothermal treatment device 2 comes into contact with steam supplied to the first hydrothermal treatment device 2. The contacted steam condenses into hot water, and hydrolysis reaction occurs in the waste in contact with the hot water. The type of waste is not limited, but may include plant waste containing cellulose as one component, such as palm residue, and animal waste containing protein as one component, such as livestock manure and food waste such as meat and fish. Further, the waste may include municipal waste, such as paper waste and food residue. The conditions for hydrothermal treatment may be, for example, a temperature between 120°C and 240°C, a pressure between 0.2 MPa and 3.4 MPa, which is saturation pressure, and a treatment time between 15 minutes and 2 hours, but the conditions are not limited thereto and can be adjusted according to the type of waste.

The first reactant which is a reactant of the hydrothermal treatment in the first hydrothermal treatment device 2 is separated by the first solid-liquid separation device 3 into a solid and a liquid. The separated solid is sent to the second hydrothermal treatment device 12, comes into contact with steam supplied to the second hydrothermal treatment device 12, and is hydrothermally treated using the same principle as the hydrothermal treatment in the first hydrothermal treatment device 2. The temperature of the hydrothermal treatment in the second hydrothermal treatment device 12 may be higher than the temperature of the hydrothermal treatment in the first hydrothermal treatment device 2. For example, preferably, the temperature of the hydrothermal treatment in the first hydrothermal treatment device 2 is 120°C to 160°C, and the temperature of the hydrothermal treatment in the second hydrothermal treatment device 12 is 200°C to 240°C.

The second reactant which is a reactant of the hydrothermal treatment in the second hydrothermal treatment device 12 is separated by the second solid-liquid separation device 13 into a solid and a liquid. The separated solid is those that cannot be decomposed by the action of microorganisms and bacteria in the fermentation device 6, such as plastic, metal, stone, sand, etc., and is disposed of as a material unsuitable for fermentation that cannot be used as the raw material for fermentation in the fermentation device 6. Alternatively, of the material unsuitable for fermentation, valuable metal and plastic that can be used as fuel are recycled as resources. On the other hand, the liquids separated by the first solid-liquid separation device 3 and the second solid-liquid separation device 13 are sent to the fermentation device 6 to produce a fermented substance such as biogas, ethanol, organic fertilizer, and feed.

When organic waste containing saccharides and proteins is hydrothermally treated at a temperature of 150°C or higher, melanoidin is generated by the Maillard reaction. Nitrogen-containing antioxidants such as melanoidin entering the fermentation device 6 inhibit fermentation in the fermentation device 6. In the waste treatment system 1 according to the first embodiment, in the first hydrothermal treatment device 2, the hydrothermal treatment is performed at a lower temperature than the temperature of the hydrothermal treatment in the second hydrothermal treatment device 12, preferably at a temperature that suppresses the Maillard reaction, for example at 120°C, to suppress the generation of melanoidin in the first hydrothermal treatment device 2. Further, the first hydrothermal treatment device 2 solubilizes nitrogen-containing components, which may cause melanoidin formation, and transfers them to the liquid. This reduces the nitrogen-containing components in the solid hydrothermally treated in the second hydrothermal treatment device 12, and suppresses the generation of melanoidin due to the hydrothermal treatment in the second hydrothermal treatment device 12. Thus, it is possible to reduce the fermentation inhibition in the fermentation device 6.

In the second hydrothermal treatment device 12, the hydrothermal treatment is performed at a higher temperature than the hydrothermal treatment in the first hydrothermal treatment device 2, for example at 220°C, to degrade persistent organic matter such as cellulose to easily degradable organic matter such as saccharide and organic acid. Although persistent organic matter such as cellulose takes a long time to ferment in the fermentation device 6, since the persistent organic matter such as cellulose is degraded into easily degradable organic matter by the hydrothermal treatment in the second hydrothermal treatment device 12 and supplied to the fermentation device 6, the fermentation time in the fermentation device 6 can be shortened. Further, when lignocellulosic biomass, such as grasses and paper made from grasses, is hydrothermally treated in the second hydrothermal treatment device 12, lignin decomposes and yields phenols. Phenols entering the fermentation device inhibit fermentation. Especially when the hydrothermal temperature is 240°C or higher, the decomposition of lignin is accelerated and saccharides produced by the decomposition of cellulose are polycondensed into carbides or precursors thereof. Since carbide is not used as the raw material for fermentation, the temperature of the hydrothermal treatment in the second hydrothermal treatment device 12 is preferably 200°C to 240°C.

Thus, when the hydrothermal treatment in the first hydrothermal treatment device 2 is performed at a temperature that suppresses the Maillard reaction, the generation of melanoidin in the first hydrothermal treatment device 2 can be suppressed. Further, since the first solid-liquid separation device 3 solubilizes or slurries nitrogen compounds such as proteins, which may cause melanoidin formation, and transfers them into a liquid of the first reactant, the amount of nitrogen compounds in the raw material of the second hydrothermal treatment device 12 can be reduced, and the generation of melanoidin due to the hydrothermal treatment in the second hydrothermal treatment device 12 can be suppressed. Thus, it is possible to reduce the fermentation inhibition in the fermentation device 6. As a result, it is possible to increase a treatable waste range and appropriately produce a fermented substance from treated waste without inhibition.

The waste treatment system 1 may include colored substance sensors 15 and 16 for detecting the index of a colored substance in the liquids separated by the first solid-liquid separation device 3 and the second solid-liquid separation device 13. The colored substance includes, in addition to melanoidin, phenols and furals, which can inhibit fermentation in the fermentation device 6. Since the concentration of melanoidin in the liquid correlates with the index of absorbance in the wavelength region of 400 to 450 nm, and the concentration of phenols in the liquid correlates with the index of absorbance in the wavelength region of 200 to 300 nm, absorbance meters can be used as the colored substance sensors 15, 16. Instead of the colored substance sensors 15, 16, the system may include a colored substance sensor 17 for detecting the index of the colored substance in a liquid after joining the liquids separated by the first solid-liquid separation device 3 and the second solid-liquid separation device 13 and before flowing into the fermentation device 6. In addition, a colored substance sensor 18 may be provided in the fermentation device 6 to detect the index of the colored substance in the fermentation device 6. That is, the colored substance sensors 15 to 18 are used to detect the colored substance in the liquid flowing into the fermentation device 6.

If the amount of colored substance, for example, melanoidin, flowing into the fermentation device 6 is determined to be high based on the detected values by the colored substance sensors 15 to 17, the cause may be, for example, that low solid decomposition rate in the first hydrothermal treatment device 2 causes nitrogen-containing substances to flow into the second hydrothermal treatment device 12 and melanoidin to be generated in the second hydrothermal treatment device 12. If the colored substance sensors 15 and 16 are provided, this phenomenon can be identified by comparing the detected values of both sensors. In this case, increasing the temperature of the hydrothermal treatment in the first hydrothermal treatment device 2 or increasing the residence time of the waste in the first hydrothermal treatment device 2 promotes the shrinkage of the waste and improves the solid decomposition rate, thus reducing the amount of melanoidin flowing into the fermentation device 6. However, if the temperature of the hydrothermal treatment in the first hydrothermal treatment device 2 is excessively increased, melanoidin generated in the first hydrothermal treatment device 2 increases. Therefore, the temperature of the hydrothermal treatment in the first hydrothermal treatment device 2 should be adjusted below the temperature at which melanoidin is not generated in the first hydrothermal treatment device 2. In addition, a camera or video and a light to photograph or record the inside of the first hydrothermal treatment device 2 may be provided to allow the user to check the improved shrinkage of the waste.

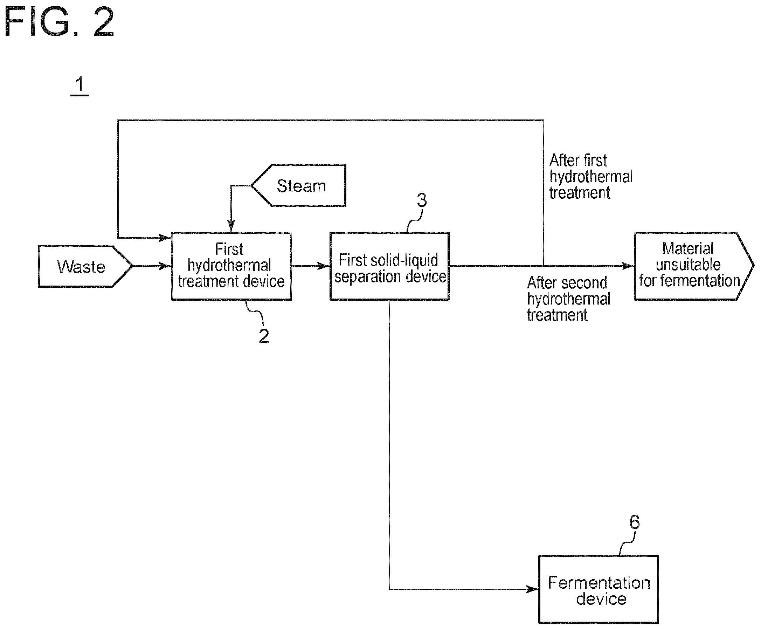

In the first embodiment, two hydrothermal treatment devices, i.e., the first hydrothermal treatment device and the second hydrothermal treatment device are provided, and two hydrothermal treatments are performed on the waste by different hydrothermal treatment devices, but the embodiment is not limited thereto. As shown in FIG. 2, the second hydrothermal treatment device 12 and the second solid-liquid separation device 13 may be eliminated from the configuration shown in FIG. 1. In this configuration, after the hydrothermal treatment in the first hydrothermal treatment device 2 (first hydrothermal treatment step), the first reactant is separated into a solid and a liquid in the first solid-liquid separation device 3 (first solid-liquid separation step). The liquid of the first reactant is sent to the fermentation device 6, while the solid is returned to the first hydrothermal treatment device 2. Then, the solid of the first reactant is hydrothermally treated in the first hydrothermal treatment device 2 (second hydrothermal treatment step), and the second reactant, which is a reactant of this hydrothermal treatment, is separated into a solid and a liquid in the first solid-liquid separation device 3 (second solid-liquid separation step). The liquid of the second reactant is sent to the fermentation device 6, while the solid is disposed of as a material unsuitable for fermentation. The fermentation of liquid in the fermentation device 6 (fermentation step) is the same as the fermentation step described in the configuration of FIG. 1. With this configuration and method, since the waste can be treated by one hydrothermal treatment device (first hydrothermal treatment device 2), the waste treatment system 1 can be downsized.

### (Second Embodiment)

Next, the waste treatment system according to the second embodiment will be described. The waste treatment system according to the second embodiment is configured such that washing water is supplied to the first solid-liquid separation device 3 and the second solid-liquid separation device 13 in contrast to the first embodiment. In the second embodiment, the same constituent elements as those in the first embodiment are associated with the same reference numerals and not described again in detail.

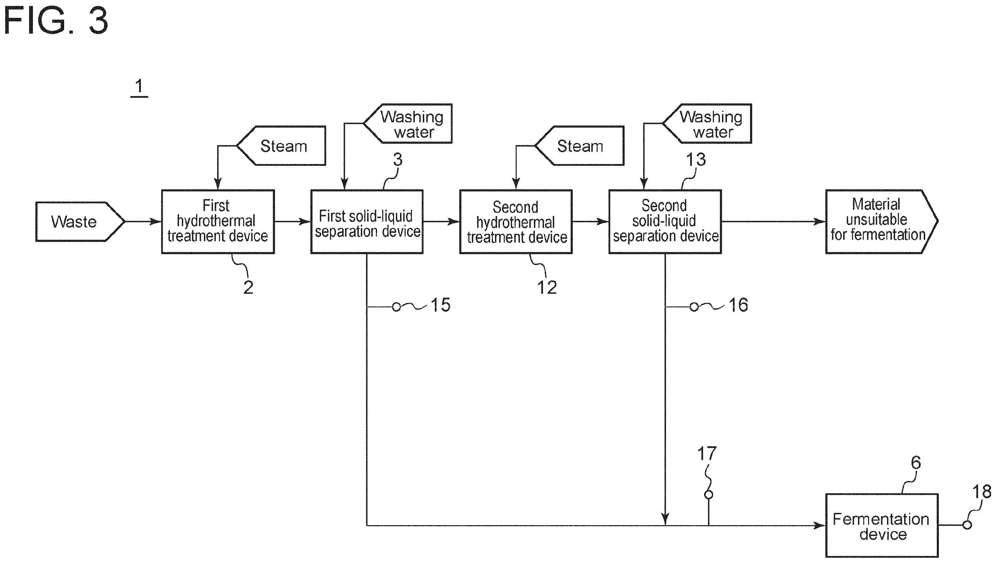

As shown in FIG. 3, the waste treatment system 1 according to the second embodiment of the present disclosure is configured such that washing water is supplied to the first solid-liquid separation device 3 and the second solid-liquid separation device 13. The configuration is otherwise the same as that of the first embodiment.

The operation of hydrothermal treatment of waste in the first hydrothermal treatment device 2 of the waste treatment system 1 according to the second embodiment of the present disclosure is the same as the first embodiment. Then, the first reactant of the first hydrothermal treatment device 2 is separated into a solid and a liquid by the first solid-liquid separation device 3, but water adheres to or is contained in the solid separated by the first solid-liquid separation device 3. If this water contains saccharides and proteins, the hydrothermal treatment in the second hydrothermal treatment device 12 may produce melanoidin. However, in the second embodiment, since the solid is washed by washing water during solid-liquid separation in the first solid-liquid separation device 3, compared to the case without washing, the saccharides and proteins contained in the separated solid can be reduced, and as a result, melanoidin that can be generated by the hydrothermal treatment in the second hydrothermal treatment device 12 can be reduced.

If the waste treatment system 1 includes the colored substance sensors 15 to 18, and it is determined that the amount of melanoidin flowing into the fermentation device 6 is high based on the detected values, the flow rate of washing water supplied to the first solid-liquid separation device 3 may be increased. When the flow rate of washing water is increased, the washing of the solid during solid-liquid separation in the first solid-liquid separation device 3 is enhanced, so that more nitrogen-containing components, which may cause melanoidin formation, can be transferred to the liquid. This reduces the nitrogen-containing components in the solid hydrothermally treated in the second hydrothermal treatment device 12, and thus appropriately reduces melanoidin that can be generated due to the hydrothermal treatment in the second hydrothermal treatment device 12. Further, other effects such as simply reducing the concentration of inhibitors can also be expected by increasing the flow rate of washing water.

The solid separated by the second solid-liquid separation device 13 is disposed of as a material unsuitable for fermentation. However, since the solid separated by the second solid-liquid separation device 13 has moisture attached or contained therein, if this moisture contains easily degradable organic matter, the easily degradable organic matter that should be used as the raw material for fermentation in the fermentation device 6 is disposed of together with the solid such as metal and plastic, and the yield of the fermented substance is reduced. However, in the second embodiment, since the solid is washed by washing water during solid-liquid separation in the second solid-liquid separation device 13, compared to the case without washing, the easily degradable organic matter supplied to the fermentation device 6 can be increased, and the yield of the fermented substance can be improved.

### (Third Embodiment)

Next, the waste treatment system according to the third embodiment will be described. The waste treatment system according to the third embodiment is configured such that waste is divided and supplied to the first hydrothermal treatment device 2 and the second hydrothermal treatment device 12 in contrast to the first or second embodiment. In the following, the third embodiment will be described in conjunction with the configuration where waste is divided and supplied based on the second embodiment, but the third embodiment may be implemented with the configuration where waste is divided and supplied based on the first embodiment. In the third embodiment, the same constituent elements as those in the second embodiment are associated with the same reference numerals and not described again in detail.

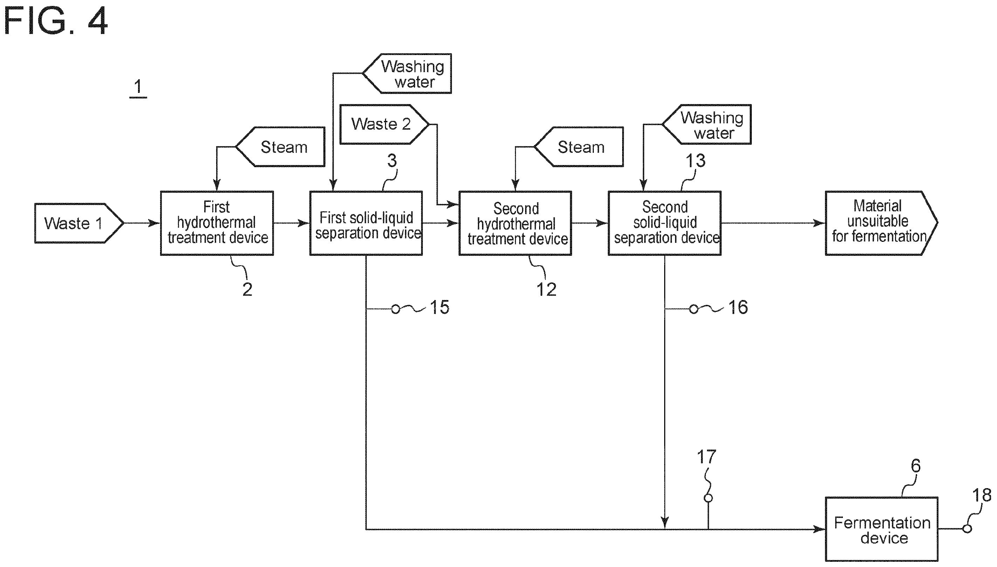

As shown in FIG. 4, the waste treatment system 1 according to the third embodiment of the present disclosure is configured such that waste can be supplied to the first hydrothermal treatment device 2 and the second hydrothermal treatment device 12, separately. The configuration is otherwise the same as that of the second embodiment.

In the third embodiment, for example, waste 2 containing a large amount of persistent fibers (e.g., paper such as used paper or recycled paper, biomass containing a large amount of fiber material) can be supplied to the second hydrothermal treatment device 12, while waste 1 other than waste 2 can be supplied to the first hydrothermal treatment device 2. In other words, the waste can be divided and supplied separately to the first hydrothermal treatment device 2 and the second hydrothermal treatment device 12 according to the type. Since the waste 2 contains few nitrogen-containing substances, the waste 2 does not need to undergo hydrothermal treatment at a relatively low temperature in the first hydrothermal treatment device 2. On the other hand, when the waste 2 that only needs to be hydrothermally treated at a relatively high temperature to refine and organically oxidize the fibers is supplied to the second hydrothermal treatment device 12, the efficiency of the hydrothermal treatment in each of the first hydrothermal treatment device 2 and the second hydrothermal treatment device 12 can be improved.

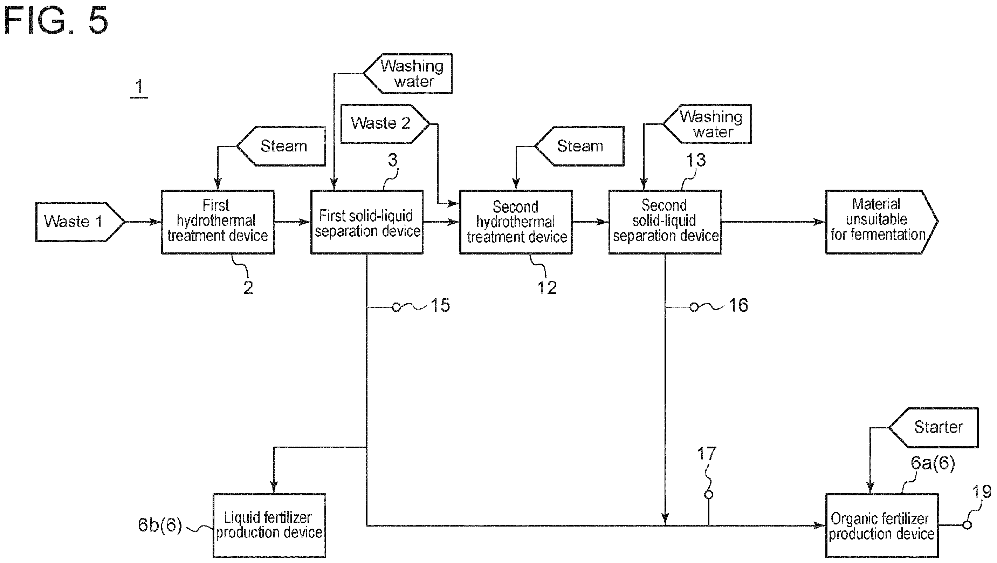

For example, FIG. 5 shows the waste treatment system 1 in which waste containing proteins (nitrogen-containing substances) such as food waste (livestock manure, methane fermentation residue, palm oil mill effluent (POME) residue, etc.) is supplied to the first hydrothermal treatment device 2 as the waste 1, while palm empty fruit bunch (FEB) is supplied to the second hydrothermal treatment device 12 as the waste 2, and the liquids of the first and second reactants are fermented in an organic fertilizer production device 6a as the fermentation device 6 to produce an organic fertilizer. The organic fertilizer production device 6a is configured to add a starter (e.g., anti-fungal bacteria). The organic fertilizer production device 6a may also be equipped with a colored substance sensor 19.

Fermentation in the organic fertilizer production device 6a preferably allows the added anti-fungal bacteria to grow appropriately. If the liquid received by the organic fertilizer production device 6a contains waste-derived germs, these germs may inhibit the growth of the anti-fungal bacteria and prevent the fermentation from progressing appropriately. However, since the liquid received by the organic fertilizer production device 6a is part of the first reactant and the second reactant hydrothermally treated in the first hydrothermal treatment device 2 and the second hydrothermal treatment device 12 respectively, the germs in the wastes 1 and 2 are sterilized during the hydrothermal treatments in the first hydrothermal treatment device 2 and the second hydrothermal treatment device 12. In the organic fertilizer production device 6a, since the anti-fungal bacteria are added to the liquid where germs have been sterilized, the anti-fungal bacteria can appropriately multiply during fermentation, and the fermentation can proceed appropriately. Further, the first hydrothermal treatment device 2 is expected to solubilize nitrogen-containing components in the waste 1, and the second hydrothermal treatment device 12 is expected to decompose fiber materials in the waste 2.

The ratio of carbon components to nitrogen components (C/N ratio) is important as the index of the composition of a good organic fertilizer produced by the organic fertilizer production device 6a, and the C/N ratio is preferably 40 or less. The nitrogen component is mainly present in the liquid separated by the first solid-liquid separation device 3 from the waste 1 decomposed in the first hydrothermal treatment device 2, while the carbon component is mainly present in the liquid separated by the second solid-liquid separation device 13 from the solid of the first reactant and the waste 2 decomposed in the second hydrothermal treatment device 12. Therefore, by supplying the necessary amount of the liquid separated by the first solid-liquid separation device 3 to the organic fertilizer production device 6a, the C/N ratio can be adjusted to 40 or less.

If not all of the liquid separated by the first solid-liquid separation device 3 is supplied to the organic fertilizer production device 6a in order to adjust the C/N ratio, the waste treatment system 1 may be equipped with a liquid fertilizer production device 6b as the fermentation device 6 to produce a liquid fertilizer from the remaining liquid.

### (Fourth Embodiment)

Next, the waste treatment system according to the fourth embodiment will be described. In the waste treatment system according to the fourth embodiment, the fermentation device 6 is limited to a methane fermentation device for producing biogas in contrast to the first embodiment. In the fourth embodiment, the same constituent elements as those in the first embodiment are associated with the same reference numerals and not described again in detail.

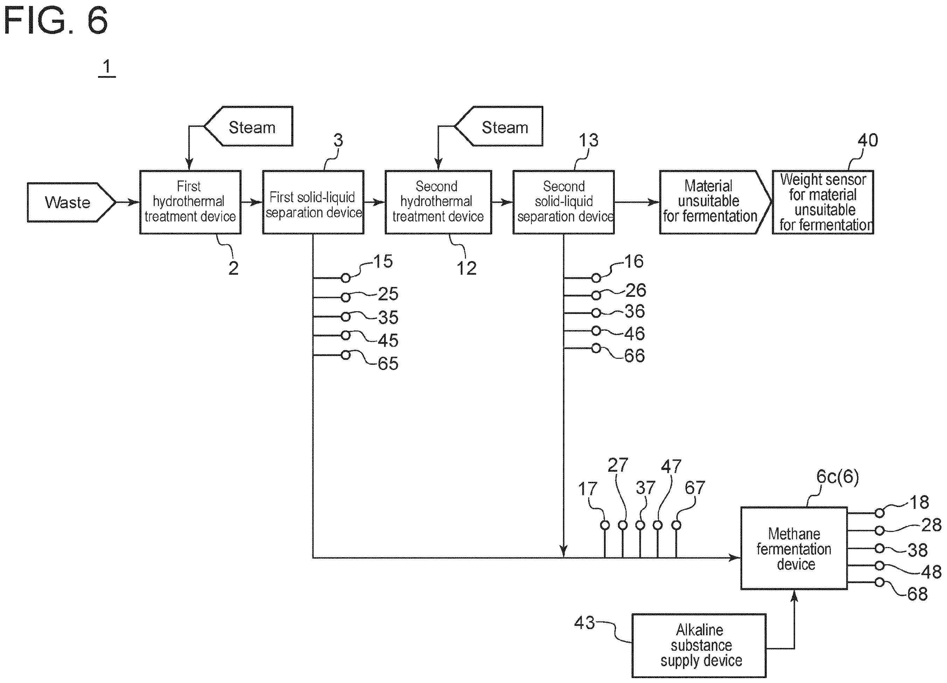

As shown in FIG. 6, in the waste treatment system 1 according to the fourth embodiment of the present disclosure, the fermentation device 6 is a methane fermentation device 6c for producing biogas. The configuration of the methane fermentation device 6c is not limited. For example, it may be provided with a fermentation tank for receiving enzymes and bacteria, e.g., methane fermentation bacteria to decompose organic matter in an anaerobic environment. The methane fermentation device 6c may be equipped with a purification device for purifying the produced biogas to obtain methane gas, and a storage facility for storing the produced biogas or methane gas. Although not a required component, the waste treatment system 1 may also include an alkaline substance supply device 43 for supplying an alkaline substance to the methane fermentation device 6c. As the alkaline substance, any liquid or solid substance with alkaline properties greater than pH 7 can be used. The configuration is otherwise the same as that of the first embodiment.

Although not a required component, the waste treatment system 1 according to the fourth embodiment of the present disclosure may include colored substance sensors 15 and 16 for detecting the index of the colored substance in the liquids separated by the first solid-liquid separation device 3 and the second solid-liquid separation device 13. Instead of the colored substance sensors 15, 16, the system may include a colored substance sensor 17 for detecting the index of the colored substance in a liquid after joining the liquids separated by the first solid-liquid separation device 3 and the second solid-liquid separation device 13 and before flowing into the methane fermentation device 6c. In addition, a colored substance sensor 18 may be provided in the methane fermentation device 6c to detect the index of the colored substance in the methane fermentation device 6c.

Although not a required component, the waste treatment system 1 according to the fourth embodiment of the present disclosure may include pH sensors 25 and 26 for detecting the pH of the liquids separated by the first solid-liquid separation device 3 and the second solid-liquid separation device 13. Instead of the two pH sensors 25, 26, the system may include a pH sensor 27 for detecting the pH of a liquid after joining the liquids separated by the first solid-liquid separation device 3 and the second solid-liquid separation device 13 and before flowing into the methane fermentation device 6c. In addition, a pH sensor 28 may be provided in the methane fermentation device 6c to detect the pH in the methane fermentation device 6c.

Although not a required component, the waste treatment system 1 according to the fourth embodiment of the present disclosure may include sodium sensors 35 and 36 for detecting the concentration of sodium in the liquids separated by the first solid-liquid separation device 3 and the second solid-liquid separation device 13. Instead of the two sodium sensors 35, 36, the system may include a sodium sensor 37 for detecting the concentration of sodium in a liquid after joining the liquids separated by the first solid-liquid separation device 3 and the second solid-liquid separation device 13 and before flowing into the methane fermentation device 6c. In addition, a sodium sensor 38 may be provided in the methane fermentation device 6c to detect the concentration of sodium in the methane fermentation device 6c.

Although not a required component, the waste treatment system 1 according to the fourth embodiment of the present disclosure may include organic acid concentration sensors 45 and 46 for detecting the concentration of organic acid in the liquids separated by the first solid-liquid separation device 3 and the second solid-liquid separation device 13. Instead of the two organic acid concentration sensors 45, 46, the system may include an organic acid concentration sensor 47 for detecting the concentration of organic acid in a liquid after joining the liquids separated by the first solid-liquid separation device 3 and the second solid-liquid separation device 13 and before flowing into the methane fermentation device 6c. In addition, an organic acid concentration sensor 48 may be provided in the methane fermentation device 6c to detect the concentration of organic acid in the methane fermentation device 6c.

Although not a required component, the waste treatment system 1 according to the fourth embodiment of the present disclosure may include ammonia concentration sensors 65 and 66 for detecting the concentration of ammonia in the liquids separated by the first solid-liquid separation device 3 and the second solid-liquid separation device 13. Instead of the two ammonia concentration sensors 65, 66, the system may include an ammonia concentration sensor 67 for detecting the concentration of ammonia in a liquid after joining the liquids separated by the first solid-liquid separation device 3 and the second solid-liquid separation device 13 and before flowing into the methane fermentation device 6c. In addition, an ammonia concentration sensor 68 may be provided in the methane fermentation device 6c to detect the concentration of ammonia in the methane fermentation device 6c.

Next, operation of the waste treatment system 1 according to the fourth embodiment of the present disclosure will be described.

The operation of hydrothermal treatment in the first hydrothermal treatment device 2 and the second hydrothermal treatment device 12 and the operation of solid-liquid separation in the first solid-liquid separation device 3 and the second solid-liquid separation device 13 are the same as the first embodiment. In the fourth embodiment, the liquid of the first reactant and the liquid of the second reactant are separately supplied to the methane fermentation device 6c.

In the methane fermentation device 6c, methane fermentation of the liquid of the first reactant and the liquid of the second reactant produces biogas. Methane fermentation can be performed under anaerobic conditions, for example, between 30°C and 60°C, preferably between 50°C and 60°C for high-temperature methane fermentation using bacteria that act at high temperatures, or between 30°C and 40°C for medium-temperature methane fermentation using bacteria that act at medium temperatures. The produced biogas may be purified to obtain methane gas, and the biogas or methane gas may be stored in a storage facility.

In the case where the waste treatment system 1 according to the fourth embodiment of the present disclosure includes the colored substance sensors 15 to 18, as with the operation in the first embodiment, the amount of colored substance flowing into the methane fermentation device 6c can be reduced based on the detected values by the colored substance sensors 15 to 18.

When the pH of the liquid flowing into the methane fermentation device 6c decreases, and the supply amount of organic acid is greater than the amount of organic acid that can be decomposed in the methane fermentation device 6c, the pH in the methane fermentation device 6c decreases, and a phenomenon called rancidification, in which the production amount of biogas decreases or stops, occurs. In response, by supplying an appropriate amount of alkaline substance at an appropriate timing from the alkaline substance supply device 43 based on the detected value by the pH sensor 28, the pH of the liquid can be increased, and the phenomenon where the biogas production decreases or stops can be suppressed.

Generally, a factor that decreases the pH of the liquid is the excessive amount of organic acid produced by the hydrothermal treatment of at least one of the first hydrothermal treatment device 2 or the second hydrothermal treatment device 12. If the pH sensors 25, 26 and the organic acid concentration sensors 45, 46 are provided, by comparing the respective detected values of the sensors, it is possible to determine which hydrothermal treatment produces excessive organic acid. In this case, by lowering the hydrothermal temperature and shortening the residence time in the hydrothermal treatment device that is determined to produce excessive organic acid, the production of excessive organic acid can be suppressed, so that the pH of the liquid can be increased, and the phenomenon where biogas generation stops can be suppressed. In order to directly detect the phenomenon where the production amount of biogas decreases or stops, a gas chromatography or a flow meter may be provided at the gas outlet of the methane fermentation device 6c.

It is known that inhibition of methane fermentation occurs when the sodium concentration in the liquid flowing into the methane fermentation device 6c increases and the sodium concentration in the methane fermentation device 6c increases. In response, by diluting the liquid flowing into the methane fermentation device 6c by adding clean water based on the sodium concentration detected by the sodium sensors 35 to 38, the sodium concentration in the liquid can be reduced before methane fermentation inhibition occurs, so that methane fermentation inhibition can be suppressed.

However, the addition of clean water has the disadvantage of increasing the amount of wastewater discharged outside the waste treatment system 1. Therefore, it is preferable to add clean water while lowering the temperature of the hydrothermal treatment in the second hydrothermal treatment device 12 to shorten the residence time. When the temperature of the hydrothermal treatment in the second hydrothermal treatment device 12 is lowered to shorten the residence time, the organic oxidation and saccharification in the second hydrothermal treatment device 12 are suppressed, and with a large amount of solid residue in the reactant flowing out of the second hydrothermal treatment device 12, the liquid separated by the second solid-liquid separation device 13 is supplied to the methane fermentation device 6c. This reduces the decomposition rate of methane fermentation, resulting in a larger amount of solids discharged from the methane fermentation device 6c and a larger amount of dehydrated sludge discharged outside the waste treatment system 1. As a result, the amount of wastewater discharged outside the waste treatment system 1 can be suppressed. In order to confirm the suppression of organic oxidation in the second hydrothermal treatment device 12, the organic acid concentration sensor 46 may be provided together with the sodium sensor 36.

If compositional changes occur in waste, for example, if the caloric value of waste decreases or if ash or plastic components in waste increases, the amount of material unsuitable for fermentation to be disposed of increases, and the production amount of biogas decreases. In response, a weight sensor 40 for material unsuitable for fermentation may be provided in the waste treatment system 1. If it is judged that ash or plastic components in waste increases based on an increase in the detected value by the weight sensor 40 for material unsuitable for fermentation, the supply amount of waste may be increased to suppress the decrease in the production amount of biogas.

The hydrothermal reaction decomposes nitrogen-containing organic matter in waste to produce ammonia. Since ammonia is an inhibitor of methane fermentation, the ammonia concentration may be reduced by, for example, increasing the supply amount of paper that does not contain nitrogen-containing organic matter to the hydrothermal treatment device based on the ammonia concentration detected by the ammonia concentration sensors 65 to 68. Alternatively, the ammonia concentration may be reduced by adding diluted water to the liquid flowing into the methane fermentation device 6c, or by increasing the amount of washing water supplied to the solid-liquid separation device.

As for the sensors installed in the waste treatment system 1, if continuous or direct detection is impossible, so-called soft sensors may be used, which allow estimation by calculation from one or more other detected values.

### (Fifth Embodiment)

Next, the waste treatment system according to the fifth embodiment will be described. In the waste treatment system according to the fifth embodiment, the fermentation device 6 includes both a methane fermentation device and an organic fertilizer production device, in contrast to the fourth embodiment. In the fifth embodiment, the same constituent elements as those in the fourth embodiment are associated with the same reference numerals and not described again in detail.

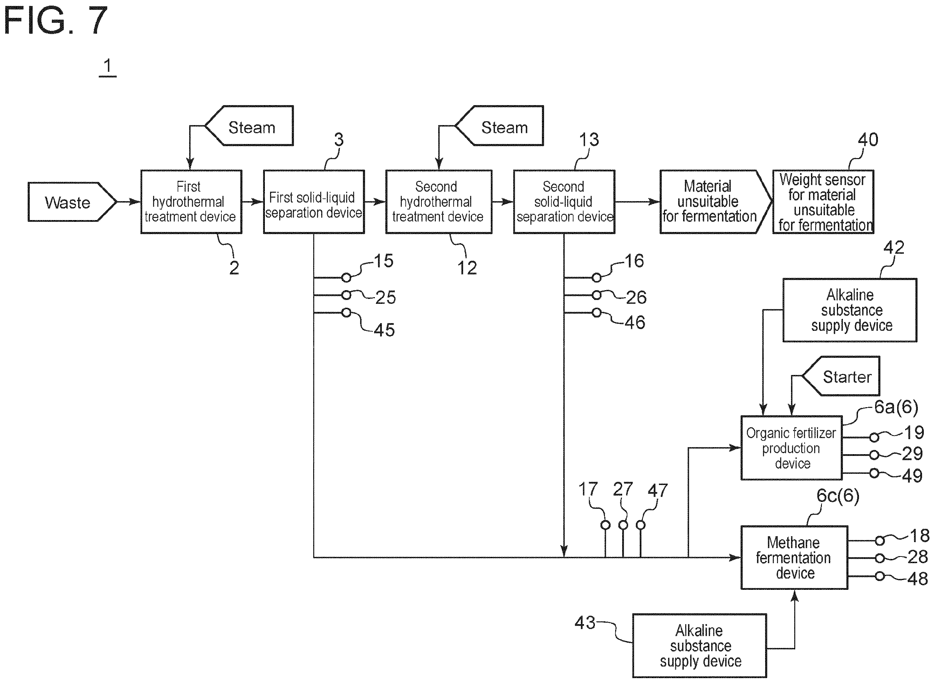

As shown in FIG. 7, in the waste treatment system 1 according to the fifth embodiment of the present disclosure, the fermentation device 6 includes a methane fermentation device 6c and an organic fertilizer production device 6a. The liquids separated by the first solid-liquid separation device 3 and the second solid-liquid separation device 13 are joined and then distributed to the methane fermentation device 6c and the organic fertilizer production device 6a. The organic fertilizer production device 6a is configured to add a starter (e.g., anti-fungal bacteria). The waste treatment system 1 may further include an alkaline substance supply device 42 for supplying an alkaline substance to the organic fertilizer production device 6a. The organic fertilizer production device 6a may be equipped with a colored substance sensor 19, a pH sensor 29, and an organic acid concentration sensor 49. The configuration is otherwise the same as that of the fourth embodiment.

In the fifth embodiment, the operation of hydrothermally treating waste and producing biogas in the methane fermentation device 6c is the same as the fourth embodiment. Further, the operation of fermenting the liquids separated by the first solid-liquid separation device 3 and the second solid-liquid separation device 13 and producing an organic fertilize in the organic fertilizer production device 6a is the same as the third embodiment (see the configuration of FIG. 5).

In the fifth embodiment, as well as fermentation in the methane fermentation device 6c, fermentation in the organic fertilizer production device 6a stops when the pH of the liquid flowing into the organic fertilizer production device 6a decreases. In response, by supplying an appropriate amount of alkaline substance at an appropriate timing from the alkaline substance supply device 42 based on the detected value by the pH sensor 29, the pH of the liquid can be increased, and the phenomenon where the fermentation stops in the organic fertilizer production device 6a can be suppressed.

### (Sixth Embodiment)

Next, the waste treatment system according to the sixth embodiment will be described. The waste treatment system according to the sixth embodiment is additionally provided with a boiler for combusting residue of methane fermentation in the methane fermentation device 6c, in contrast to the fifth embodiment. In the sixth embodiment, the same constituent elements as those in the fifth embodiment are associated with the same reference numerals and not described again in detail.

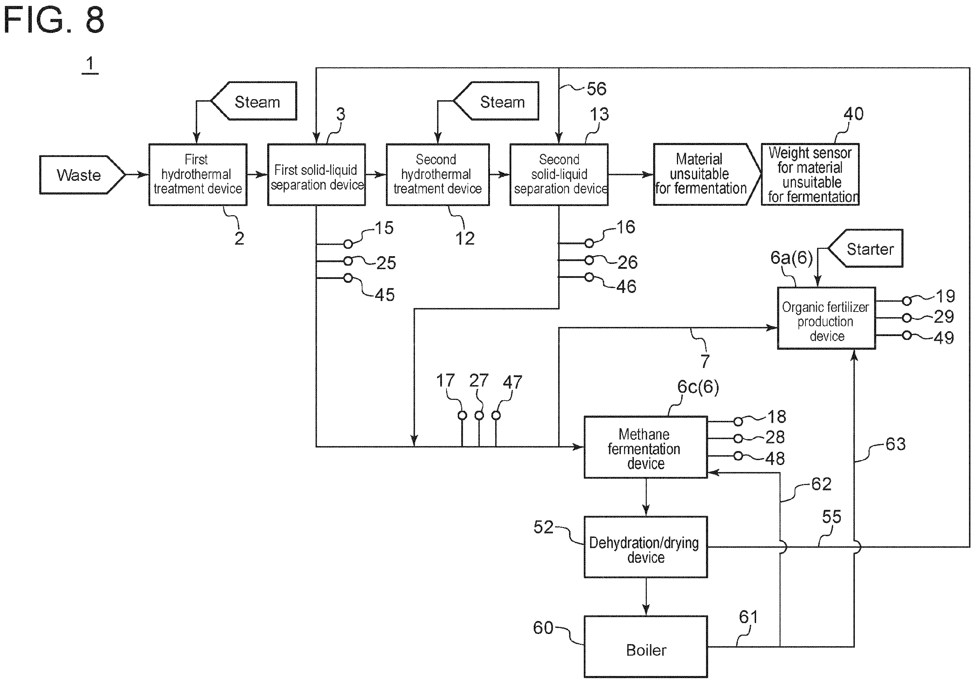

As shown in FIG. 8, the waste treatment system 1 according to the sixth embodiment of the present disclosure includes a dehydration/drying device 52 (e.g., combination of filtration device or centrifugal separation device and drying device) for dehydrating and drying residue of methane fermentation in the methane fermentation device 6c, and a boiler 60 for combusting a solid component obtained by dehydration of residue of methane fermentation by the dehydration/drying device 52. The dehydration/drying device 52 is provided with a washing water supply line 55 for supplying a liquid component obtained by dehydration of residue of methane fermentation to the first solid-liquid separation device 3 as washing water. A branch line 56 for supplying part of the liquid component as washing water to the second solid-liquid separation device 13 may branch from the washing water supply line 55. The boiler 60 is connected to an extraction line 61 for extracting ash after combustion. The extraction line 61 branches into a first ash supply line 62 communicating with the methane fermentation device 6c and a second ash supply line 63 communicating with the organic fertilizer production device 6a. The configuration is otherwise the same as that of the fifth embodiment.

The operation of producing organic fertilizer and biogas in the waste treatment system 1 according to the sixth embodiment of the present disclosure is the same as the fifth embodiment. In the waste treatment system 1 according to the sixth embodiment of the present disclosure, the solid component obtained by dehydration of residue of methane fermentation by the dehydration/drying device 52 is combusted in the boiler 60. Since ash after combustion in the boiler 60 is alkaline, the ash extracted from the boiler 60 into the extraction line 61 is supplied as an alkaline substance to the methane fermentation device 6c or the organic fertilizer production device 6a via the first ash supply line 62 or the second ash supply line 63. The presence or absence of ash supply and the amount of ash supplied to each can be adjusted appropriately based on the detected value by at least one of the pH sensors 25 to 27.

The liquid component produced by dehydration of residue of methane fermentation by the dehydration/drying device 52 is supplied as washing water to the first solid-liquid separation device 3 and the second solid-liquid separation device 13 via the washing water supply line 55 and the branch line 56, so that the solids can be washed during the solid-liquid separation in the first solid-liquid separation device 3 and the second solid-liquid separation device 13. As already described in the second embodiment, in this case, it is not necessary to prepare water separately as washing water, so the operating cost of the waste treatment system 1 can be reduced.

In the sixth embodiment, steam produced by the boiler 60 may be used as the steam supplied to each or either one of the first hydrothermal treatment device 2 and the second hydrothermal treatment device 12.

### (Seventh Embodiment)

Next, the waste treatment system according to the seventh embodiment will be described. In the waste treatment system according to the seventh embodiment, the second hydrothermal treatment device 12 is modified to a pulper in contrast to the first embodiment. In the seventh embodiment, the same constituent elements as those in the first embodiment are associated with the same reference numerals and not described again in detail.

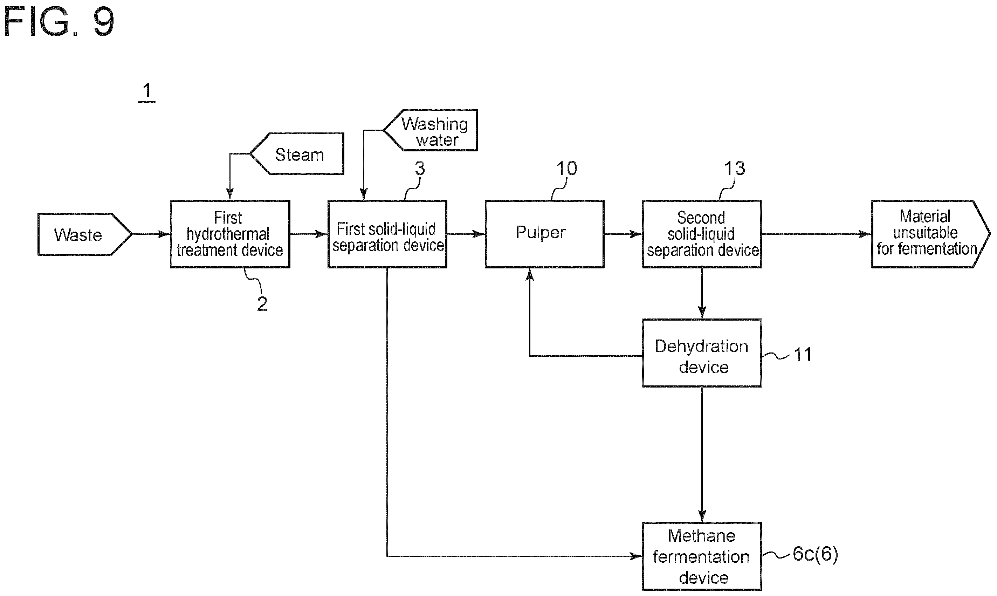

As shown in FIG. 9, in the waste treatment system 1 according to the seventh embodiment of the present disclosure, a pulper 10 is disposed between the first hydrothermal treatment device 2 and the second solid-liquid separation device 13. The pulper 10 is a device for breaking apart, or disentangling, the fibers in the solid flowing into the pulper 10. The configuration of the pulper 10 is not limited, but may be, for example, a vertical batch type with a rotor at the bottom to agitate the fluid inside. The configuration of the second solid-liquid separation device 13 is also not limited, but may be, for example, a screen composed of a plate or cylindrical metal member having multiple holes with appropriate size.

The waste treatment system 1 further includes a dehydration device 11 for dehydrating the liquid separated by the second solid-liquid separation device 13. The dehydration device 11 is configured such that water or hot water separated in the dehydration device 11 is supplied to the pulper 10. The liquid separated by the first solid-liquid separation device 3 and the solid component obtained by dehydration in the dehydration device 11 are supplied to the methane fermentation device 6c. The configuration is otherwise the same as that of the first embodiment, except that the second hydrothermal treatment device 12 (see FIG. 1) is not provided.

In the seventh embodiment, the operation of hydrothermal treatment of waste in the first hydrothermal treatment device 2 and solid-liquid separation of the first reactant in the first solid-liquid separation device 3 is the same as the first embodiment. The solid separated by the first solid-liquid separation device 3 is supplied to the pulper 10. Water or hot water is also supplied to the pulper 10. Fibers in the solid supplied to the pulper 10 are disentangled in water or hot water. The effluent from the pulper 10 flows into the screen, which is the second solid-liquid separation device 13, but the disentangled fibers pass through the screen. Those that cannot pass through the screen are disposed of as a material unsuitable for fermentation.

The effluent from the screen is dehydrated by the dehydration device 11, and the dehydrated water or hot water is supplied to the pulper 10 for reuse, while the dehydrated solid component is supplied to the methane fermentation device 6c. The liquid separated by the first solid-liquid separation device 3 is also supplied to the methane fermentation device 6c. The methane fermentation device 6c ferments them to produce biogas.

Thus, since the fibers in the solid separated by the first solid-liquid separation device 3 are disentangled by the pulper 10 and supplied to the methane fermentation device 6c after removing a material unsuitable fermentation by the second solid-liquid separation device 13, the fermentation and decomposition rate in the methane fermentation device 6c can be improved.

### Reference Signs List

- 1: Waste treatment system
- 2: First hydrothermal treatment device
- 3: First solid-liquid separation device
- 6: Fermentation device
- 6a: Organic fertilizer production device
- 6b: Liquid fertilizer production device
- 6c: Methane fermentation device
- 10: Pulper
- 12: Second hydrothermal treatment device
- 13: Second solid-liquid separation device
- 15: Colored substance sensor
- 16: Colored substance sensor
- 17: Colored substance sensor
- 18: Colored substance sensor
- 19: Colored substance sensor
- 25: pH sensor
- 26: pH sensor
- 27: pH sensor
- 28: pH sensor
- 29: pH sensor
- 35: Sodium sensor
- 36: Sodium sensor
- 37: Sodium sensor
- 38: Sodium sensor
- 40: Weight sensor for material unsuitable for fermentation
- 42: Alkaline substance supply device
- 43: Alkaline substance supply device
- 45: Organic acid concentration sensor
- 46: Organic acid concentration sensor
- 47: Organic acid concentration sensor
- 48: Organic acid concentration sensor
- 49: Organic acid concentration sensor
- 52: Dehydration/drying device
- 55: Washing water supply line
- 56: Branch line
- 60: Boiler
- 61: Extraction line
- 62: First ash supply line
- 63: Second ash supply line
- 65: Ammonia concentration sensor
- 66: Ammonia concentration sensor
- 67: Ammonia concentration sensor
- 68: Ammonia concentration sensor

## Claims

1. A waste treatment system (1), comprising:
a first hydrothermal treatment device (2) for performing hydrothermal treatment of waste;
a first solid-liquid separation device (3) for separating a first reactant of the first hydrothermal treatment device (2) into a first separated product which is a solid and a second separated product which is a liquid or slurry;
a second hydrothermal treatment device (12) for performing hydrothermal treatment of the first separated product;
a second solid-liquid separation device (13) for separating a second reactant of the second hydrothermal treatment device (12) into a third separated product which is a solid and a fourth separated product which is a liquid or slurry; and
a fermentation device (6) for fermenting the second separated product and the fourth separated product, wherein a first temperature which is a temperature of the hydrothermal treatment in the first hydrothermal treatment device (2) is a temperature that suppresses generation of a fermentation inhibitor by Maillard reaction, and a second temperature which is a temperature of the hydrothermal treatment in the second hydrothermal treatment device (12) is a temperature that suppresses generation of a fermentation inhibitor including an aromatic compound, a carbide, or a precursor thereof from the first separated product.

2. The waste treatment system (1) according to claim 1, wherein the first temperature is 120°C to 160°C, and the second temperature is 200°C to 240°C.

3. The waste treatment system (1) according to claim 1 or 2, comprising at least one sensor for detecting a concentration of the fermentation inhibitor contained in at least one of the second separated product or the fourth separated product.

4. The waste treatment system (1) according to claim 3, wherein the at least one sensor includes at least one of:
at least one colored substance sensor (15, 16, 17, 18, 19) for detecting an index of a colored substance as the fermentation inhibitor;
at least one pH sensor (25, 26, 27, 28, 29) for detecting pH of at least one of the second separated product or the fourth separated product;
at least one sodium sensor (35, 36, 37, 38) for detecting a concentration of sodium as the fermentation inhibitor;
at least one organic acid concentration sensor (45, 46, 47, 48, 49) for detecting a concentration of an organic acid as the fermentation inhibitor; or at least one ammonia concentration sensor (65, 66, 67, 68) for detecting a concentration of ammonia as the fermentation inhibitor.

5. The waste treatment system (1) according to claim 3 or 4, wherein the at least one sensor includes at least one of: a sensor (15, 25, 35, 45, 65) for detecting a concentration of the fermentation inhibitor contained in the second separated product; a sensor (16, 26, 36, 46, 66) for detecting a concentration of the fermentation inhibitor contained in the fourth separated product; or a sensor (18, 28, 38, 48, 68) for detecting a concentration of the fermentation inhibitor in the fermentation device (6).

6. The waste treatment system (1) according to any one of claims 1 to 5, wherein the second hydrothermal treatment device (12) is configured to be supplied with part of the waste.

7. The waste treatment system (1) according to any one of claims 1 to 6, wherein each of the first solid-liquid separation device (3) and the second solid-liquid separation device (13) is configured to be supplied with washing water.

8. The waste treatment system (1) according to any one of claims 1 to 7, comprising an alkaline substance supply device (42, 43) for supplying an alkaline substance to the fermentation device (6).

9. The waste treatment system (1) according to any one of claims 1 to 8, wherein the fermentation device (6) includes an organic fertilizer production device (6a) for fermenting the second separated product and the fourth separated product to produce an organic fertilizer.

10. The waste treatment system (1) according to claim 9, comprising an alkaline substance supply device (42) for supplying an alkaline substance to the organic fertilizer production device (6a).

11. The waste treatment system (1) according to claim 9 or 10, wherein the fermentation device (6) includes a methane fermentation device (6c) for fermenting the second separated product and the fourth separated product to produce biogas, and wherein the waste treatment system (1) includes a boiler (60) for combusting a residue of methane fermentation in the methane fermentation device (6c).

12. A waste treatment method, comprising:
a first hydrothermal treatment step of performing hydrothermal treatment of waste; a first solid-liquid separation step of separating a first reactant of the first hydrothermal treatment step into a first separated product which is a solid and a second separated product which is a liquid or slurry;
a second hydrothermal treatment step of performing hydrothermal treatment of the first separated product;
a second solid-liquid separation step of separating a second reactant of the second hydrothermal treatment step into a third separated product which is a solid and a fourth separated product which is a liquid or slurry; and a fermentation step of fermenting the second separated product and the fourth separated product, wherein a first temperature which is a temperature of the hydrothermal treatment in the first hydrothermal treatment device is a temperature that suppresses generation of a fermentation inhibitor by Maillard reaction, and a second temperature which is a temperature of the hydrothermal treatment in the second hydrothermal treatment device is a temperature that suppresses generation of a fermentation inhibitor including an aromatic compound, a carbide, or a precursor thereof from the first separated product.

13. The waste treatment method according to claim 12, wherein the first temperature is 120°C to 160°C, and the second temperature is 200°C to 240°C.

14. The waste treatment method according to claim 12 or 13, wherein the first hydrothermal treatment step and the second hydrothermal treatment step are performed in a same hydrothermal treatment device.

15. The waste treatment method according to claim 12 or 13, wherein the second hydrothermal treatment step is performed in a different hydrothermal treatment device from a hydrothermal device that has performed the first hydrothermal treatment step.

## Patentansprüche

1. Abfallbehandlungssystem (1), umfassend:
eine erste hydrothermische Behandlungsvorrichtung (2) zum Durchführen einer hydrothermischen Behandlung von Abfall;
eine erste Fest-Flüssig-Trennungsvorrichtung (3), um einen ersten Reaktanten der ersten hydrothermalen Behandlungsvorrichtung (2) in ein erstes getrenntes Produkt, bei dem es sich um einen Feststoff handelt, und ein zweites getrenntes Produkt, bei dem es sich um eine Flüssigkeit oder Aufschlämmung handelt, zu trennen;
eine zweite hydrothermische Behandlungsvorrichtung (12) zum Durchführen einer hydrothermischen Behandlung des ersten getrennten Produkts;
eine zweite Fest-Flüssig-Trennungsvorrichtung (13), um einen zweiten Reaktanten der zweiten hydrothermalen Behandlungsvorrichtung (12) in ein drittes getrenntes Produkt, bei dem es sich um einen Feststoff handelt, und ein viertes getrenntes Produkt, bei dem es sich um eine Flüssigkeit oder Aufschlämmung handelt, zu trennen;
eine Fermentationsvorrichtung (6), um das zweite getrennte Produkt und das vierte getrennte Produkt zu fermentieren, wobei eine erste Temperatur, die eine Temperatur der hydrothermalen Behandlung in der ersten hydrothermalen Behandlungsvorrichtung (2) ist, eine Temperatur ist, die die Erzeugung eines Fermentationsinhibitors durch Maillard-Reaktion unterdrückt, und eine zweite Temperatur, die eine Temperatur der hydrothermalen Behandlung in der zweiten hydrothermalen Behandlungsvorrichtung (12) ist, eine Temperatur ist, die die Erzeugung eines Fermentationsinhibitors, einschließlich einer aromatischen Verbindung, eines Carbids oder eines Vorläufers davon, aus dem ersten separaten Produkt unterdrückt.

2. Abfallbehandlungssystem (1) nach Anspruch 1, wobei die erste Temperatur 120°C bis 160 °C und die zweite Temperatur 200 °C bis 240 °C beträgt.

3. Abfallbehandlungssystem (1) nach Anspruch 1 oder 2, umfassend mindestens einen Sensor zum Erkennen einer Konzentration des Fermentationsinhibitors, der in mindestens einem von dem zweiten getrennten Produkt oder dem vierten getrennten Produkt enthalten ist.

4. Abfallbehandlungssystem (1) nach Anspruch 3, wobei der mindestens eine Sensor mindestens eines der Folgenden einschließt:
mindestens einen Farbstoffsensor (15, 16, 17, 18, 19) zum Erkennen eines Indexes eines Farbstoffs als Fermentationsinhibitor;
mindestens einen pH-Sensor (25, 26, 27, 28, 29) zum Bestimmen des pH-Werts des zweiten oder des vierten getrennten Produkts;
mindestens einen Natriumsensor (35, 36, 37, 38) zum Erkennen einer Konzentration von Natrium als Fermentationsinhibitor;
mindestens einen Organsäure-Konzentrationssensor (45, 46, 47, 48, 49) zum Erkennen einer Konzentration einer Organsäure als Fermentationsinhibitor; oder mindestens einen Ammoniak-Konzentrationssensor (65, 66, 67, 68) zum Erkennen einer Konzentration von Ammoniak als Fermentationsinhibitor.

5. Abfallbehandlungssystem (1) nach Anspruch 3 oder 4, wobei der mindestens eine Sensor mindestens eines der Folgenden einschließt: einen Sensor (15, 25, 35, 45, 65) zum Erkennen einer Konzentration des in dem zweiten getrennten Produkt enthaltenen Fermentationsinhibitors; einen Sensor (16, 26, 36, 46, 66) zum Erkennen einer Konzentration des in dem vierten getrennten Produkt enthaltenen Fermentationsinhibitors; oder einen Sensor (18, 28, 38, 48, 68) zum Erkennen einer Konzentration des Fermentationsinhibitors in der Fermentationsvorrichtung (6).

6. Abfallbehandlungssystem (1) nach einem der Ansprüche 1 bis 5, wobei die zweite hydrothermale Behandlungsvorrichtung (12) so konfiguriert ist, dass ihr ein Teil des Abfalls zugeführt wird.

7. Abfallbehandlungssystem (1) nach einem der Ansprüche 1 bis 6, wobei sowohl die erste Fest-Flüssig-Trennungsvorrichtung (3) als auch die zweite Fest-Flüssig-Trennungsvorrichtung (13) so konfiguriert ist, dass ihr Waschwasser zugeführt wird.

8. Abfallbehandlungssystem (1) nach einem der Ansprüche 1 bis 7, umfassend eine Alkalisubstanz-Zuführvorrichtung (42, 43) zum Zuführen einer Alkalisubstanz zu der Fermentationsvorrichtung (6).

9. Abfallbehandlungssystem (1) nach einem der Ansprüche 1 bis 8, wobei die Fermentationsvorrichtung (6) eine Organdüngerherstellungsvorrichtung (6a) einschließt, um das zweite getrennte Produkt und das vierte getrennte Produkt zu fermentieren, um einen Organdünger herzustellen.

10. Abfallbehandlungssystem (1) nach Anspruch 9, umfassend eine Alkalisubstanz-Zuführvorrichtung (42) zum Zuführen einer Alkalisubstanz an die Organdüngerherstellungsvorrichtung (6a).

11. Abfallbehandlungssystem (1) gemäß Anspruch 9 oder 10, wobei die Fermentationsvorrichtung (6) eine Methanfermentationsvorrichtung (6c) zum Fermentieren des zweiten getrennten Produkts und des vierten getrennten Produkts einschließt, um Biogas herzustellen, und wobei das Abfallbehandlungssystem (1) einen Kessel (60) zum Verbrennen eines Restes der Methanfermentation in der Methanfermentationsvorrichtung (6c) einschließt.

12. Abfallbehandlungsverfahren, umfassend:
einen ersten hydrothermalen Behandlungsschritt zum Durchführen einer hydrothermalen Abfallbehandlung; einen ersten Fest-Flüssig-Trennungsschritt zum Trennen eines ersten Reaktanten aus dem ersten hydrothermalen Behandlungsschritt in ein erstes getrenntes Produkt, das ein Feststoff ist, und ein zweites getrenntes Produkt, das eine Flüssigkeit oder eine Aufschlämmung ist; einen zweiten hydrothermalen Behandlungsschritt zum Durchführen einer hydrothermalen Behandlung des ersten getrennten Produkts;
einen zweiten Fest-Flüssig-Trennungsschritt, bei dem ein zweiter Reaktant des zweiten hydrothermalen Behandlungsschritts in ein drittes getrenntes Produkt, bei dem es sich um einen Feststoff handelt, und ein viertes getrenntes Produkt, bei dem es sich um eine Flüssigkeit oder eine Aufschlämmung handelt, getrennt wird; und
einen Fermentationsschritt, um das zweite getrennte Produkt und das vierte getrennte Produkt zu fermentieren, wobei eine erste Temperatur, die eine Temperatur der hydrothermalen Behandlung in der ersten hydrothermalen Behandlungsvorrichtung ist, eine Temperatur ist, die die Erzeugung eines Fermentationsinhibitors durch Maillard-Reaktion unterdrückt, und eine zweite Temperatur, die eine Temperatur der hydrothermalen Behandlung in der zweiten hydrothermalen Behandlungsvorrichtung ist, eine Temperatur ist, die die Erzeugung eines Fermentationsinhibitors, einschließlich einer aromatischen Verbindung, eines Carbids oder eines Vorläufers davon, aus dem ersten separaten Produkt unterdrückt.

13. Abfallbehandlungsverfahren nach Anspruch 12, wobei die erste Temperatur 120 °C bis 160 °C und die zweite Temperatur 200 °C bis 240 °C beträgt.

14. Abfallbehandlungsverfahren nach Anspruch 12 oder 13, wobei der erste hydrothermale Behandlungsschritt und der zweite hydrothermale Behandlungsschritt in derselben hydrothermalen Behandlungsvorrichtung durchgeführt werden.

15. Abfallbehandlungsverfahren nach Anspruch 12 oder 13, wobei der zweite hydrothermale Behandlungsschritt in einer anderen hydrothermalen Behandlungsvorrichtung durchgeführt wird als der hydrothermale Behandlungsschritt, der den ersten hydrothermalen Behandlungsschritt durchgeführt hat.

## Revendications

1. Système de traitement des déchets (1), comprenant :
un premier dispositif de traitement hydrothermique (2) pour la réalisation d'un traitement hydrothermique des déchets ;
un premier dispositif de séparation solide-liquide (3) pour la séparation d'un premier réactif du premier dispositif de traitement hydrothermique (2) en un premier produit séparé qui est un solide et un deuxième produit séparé qui est un liquide ou une boue ;
un second dispositif de traitement hydrothermique (12) pour la réalisation d'un traitement hydrothermique du premier produit séparé ;
un second dispositif de séparation solide-liquide (13) pour la séparation d'un second réactif du second dispositif de traitement hydrothermique (12) en un troisième produit séparé qui est un solide et un quatrième produit séparé qui est un liquide ou une boue ; et
un dispositif de fermentation (6) pour la fermentation du deuxième produit séparé et du quatrième produit séparé, dans lequel une première température, qui est une température du traitement hydrothermique dans le premier dispositif de traitement hydrothermique (2), est une température qui supprime la génération d'un inhibiteur de fermentation par réaction de Maillard, et une seconde température, qui est une température du traitement hydrothermique dans le second dispositif de traitement hydrothermique (12), est une température qui supprime la génération d'un inhibiteur de fermentation comprenant un composé aromatique, un carbure ou un précurseur de ceux-ci à partir du premier produit séparé.

2. Système de traitement des déchets (1) selon la revendication 1, dans lequel la première température est de 120 °C à 160 °C, et la seconde température est de 200°C à 240°C.

3. Système de traitement des déchets (1) selon la revendication 1 ou 2, comprenant au moins un capteur pour la détection d'une concentration de l'inhibiteur de fermentation contenu dans au moins l'un du deuxième produit séparé ou du quatrième produit séparé.

4. Système de traitement des déchets (1) selon la revendication 3, dans lequel l'au moins un capteur comprend au moins l'un parmi :
au moins un capteur de substance colorée (15, 16, 17, 18, 19) pour la détection d'un indice de substance colorée comme l'inhibiteur de fermentation ;
au moins un capteur de pH (25, 26, 27, 28, 29) pour la détection d'un pH d'au moins un parmi le deuxième produit séparé ou le quatrième produit séparé ;
au moins un capteur de sodium (35, 36, 37, 38) pour la détection d'une concentration de sodium comme l'inhibiteur de fermentation ;
au moins un capteur de concentration d'acide organique (45, 46, 47, 48, 49) pour la détection d'une concentration d'acide organique comme l'inhibiteur de fermentation ;
ou au moins un capteur de concentration d'ammoniac (65, 66, 67, 68) pour la détection d'une concentration d'ammoniac comme l'inhibiteur de fermentation.

5. Système de traitement des déchets (1) selon la revendication 3 ou 4, dans lequel l'au moins un capteur comprend au moins un parmi : un capteur (15, 25, 35, 45, 65) pour la détection d'une concentration de l'inhibiteur de fermentation contenu dans le deuxième produit séparé ; un capteur (16, 26, 36, 46, 66) pour la détection d'une concentration de l'inhibiteur de fermentation contenu dans le quatrième produit séparé ; ou un capteur (18, 28, 38, 48, 68) pour la détection d'une concentration de l'inhibiteur de fermentation dans le dispositif de fermentation (6).

6. Système de traitement des déchets (1) selon l'une quelconque des revendications 1 à 5, dans lequel le second dispositif de traitement hydrothermique (12) est configuré pour être alimenté par une partie des déchets.

7. Système de traitement des déchets (1) selon l'une quelconque des revendications 1 à 6, dans lequel chacun parmi le premier dispositif de séparation solide-liquide (3) et le second dispositif de séparation solide-liquide (13) est configuré pour être alimenté en eau de lavage.

8. Système de traitement des déchets (1) selon l'une quelconque des revendications 1 à 7, comprenant un dispositif d'alimentation en substance alcaline (42, 43) pour la fourniture d'une substance alcaline au dispositif de fermentation (6).

9. Système de traitement des déchets (1) selon l'une quelconque des revendications 1 à 8, dans lequel le dispositif de fermentation (6) comprend un dispositif de production d'engrais organique (6a) pour la fermentation du deuxième produit séparé et du quatrième produit séparé afin de produire un engrais organique.

10. Système de traitement des déchets (1) selon la revendication 9, comprenant un dispositif d'alimentation en substance alcaline (42) pour la fourniture d'une substance alcaline au dispositif de production d'engrais organique (6a).

11. Système de traitement des déchets (1) selon la revendication 9 ou 10, dans lequel le dispositif de fermentation (6) comprend un dispositif de fermentation de méthane (6c) pour la fermentation du deuxième produit séparé et du quatrième produit séparé pour produire du biogaz, et dans lequel le système de traitement des déchets (1) comprend une chaudière (60) pour brûler un reste de fermentation de méthane dans la fermentation de méthane.
(6c).

12. Procédé de traitement des déchets comprenant :
une première étape de traitement hydrothermique pour la réalisation d'un traitement hydrothermique de déchets ; une première étape de séparation solide-liquide d'un premier réactif de la première étape de traitement hydrothermique en un premier produit séparé qui est un solide et un deuxième produit séparé qui est un liquide ou une boue ; une seconde étape de traitement hydrothermique d'un traitement hydrothermique du premier produit séparé ;
une seconde étape de séparation solide-liquide pour la séparation d'un second réactif de la seconde étape de traitement hydrothermique en un troisième produit séparé qui est un solide et un quatrième produit séparé qui est un liquide ou une boue ; et une étape de fermentation pour la fermentation du deuxième produit séparé et du quatrième produit séparé, dans lequel une première température, qui est une température du traitement hydrothermique dans le premier dispositif de traitement hydrothermique, est une température qui supprime la génération d'un inhibiteur de fermentation par réaction de Maillard, et une seconde température, qui est une température du traitement hydrothermique dans le second dispositif de traitement hydrothermique, est une température qui supprime la génération d'un inhibiteur de fermentation comprenant un composé aromatique, un carbure ou un précurseur de ceux-ci à partir du premier produit séparé.

13. Procédé de traitement des déchets selon la revendication 12, dans lequel la première température est de 120 °C à 160 °C, et la seconde température est de 200 °C à 240 °C.

14. Procédé de traitement des déchets selon la revendication 12 ou 13, dans lequel la première étape de traitement hydrothermique et la seconde étape de traitement hydrothermique sont réalisées dans un même dispositif de traitement hydrothermique.

15. Procédé de traitement des déchets selon la revendication 12 ou 13, dans lequel la seconde étape de traitement hydrothermique est réalisée dans un dispositif de traitement hydrothermique différent d'un dispositif hydrothermique qui a réalisé la première étape de traitement hydrothermique.
